# EUROPEAN PATENT APPLICATION

(11) **EP 3 056 142 A1**
(43) Date of publication of application: **17.08.2016**
(21) Application number: 15164274.1
(22) Date of filing: 20.04.2015
(51) Int. Cl.: A61B 5/024, A61B 5/0428, A61B 5/0452

(54) **PHYSIOLOGICAL SIGNAL PROCESSING CIRCUIT**

(30) Priority: 16.02.2015 US 201514623103
(71) Applicant: MediaTek, Inc, Hsin-Chu 300 (TW)
(72) Inventor: Ku, Po-Wen, Hsin-Chu 302 Jhubei City (TW)
(74) Representative: Wright, Howard Hugh Burnby

(57) **Abstract**

A physiological signal processing circuit includes an amplifier, an analog-to-digital converter, and a physiological characteristic detector circuit. The amplifier is configured to amplify an analog physiological signal of a user for providing an amplified signal. The analog-to-digital converter is coupled to the amplifier, and is configured to convert the amplified signal to a digital signal. The physiological characteristic detector circuit is coupled to the analog-to-digital converter, and configured to detect a physiological characteristic of the user from the digital signal so as to provide an output signal.

## Description

### BACKGROUND OF THE INVENTION

### Field of the Invention

The disclosure generally relates to a physiological signal processing circuit.

### Description of the Related Art

As technology advances, mobile electronic devices are playing an increasingly important role in people's lives. Some mobile electronic devices, such as smart sports bracelets, can automatically collect physiological data from users and transmit the data to other devices for further processing. However, low power consumption, low computational complexity and low data amount processed are some of the desirable features for mobile devices. There is a need to design a novel physiological signal processing device so as to overcome this problem.

### BRIEF SUMMARY OF THE INVENTION

In one exemplary embodiment, the disclosure is directed to a physiological signal processing circuit, comprising: an amplifier, amplifying an analog physiological signal of a user for providing an amplified signal; an analog-to-digital converter, coupled to the amplifier, and converting the amplified signal to a digital signal; and a physiological characteristic detector circuit, coupled to the analog-to-digital converter, and detecting a physiological characteristic of the user from the digital signal so as to provide an output signal.

In some embodiments, the analog physiological signal is a photoplethysmogram (PPG) signal or an electrocardiography (ECG) signal. In some embodiments, the output signal comprises one of a period between two successive heartbeats or a specific time instant with respect to a heartbeat. In some embodiments, a data rate of the output signal is lower than a data rate of the digital signal. In some embodiments, the physiological characteristic of the user is one of a heart rate, a heart beat interval, and a heart beat instant. In some embodiments, the physiological signal processing circuit further comprises: a processor; and a data storage unit for storing the output signal, wherein the processor is triggered to process the output signal stored in the data storage unit when a trigger condition occurs. In some embodiments, wherein the physiological characteristic detector circuit comprises: a filter, filtering the digital signal to provide a filtered signal in a first signal domain; and a post-filter processing circuit, process the filtered signal to provide an intermediate signal in a second signal domain. In some embodiments, the physiological characteristic detector further comprises: a peak detection circuit, detecting local peak values among the intermediate signal for providing a plurality of data points; and a decision circuit, selecting some of the plurality of data points as a plurality of heartbeat points, wherein the plurality of heartbeat points are used to produce the output signal. In some embodiments, the physiological characteristic detector circuit is further coupled to a data transmitting unit for transmitting the output signal to a device through a wired or wireless communication link. In some embodiments, the physiological characteristic detector circuit is further coupled to a data storage unit, such as static random access memory (SRAM) or dynamic random access memory (DRAM) for storing the output signal. In some embodiments, a data rate of the output signal is less than 0.03 times of a data rate of the digital signal.

In another exemplary embodiment, the disclosure is directed to a method for processing physiological signals, comprising the steps of: amplifying an analog physiological signal of a user for providing an amplified signal; converting the amplified signal to a digital signal; and detecting a physiological characteristic of the user from the digital signal so as to provide an output signal.

### BRIEF DESCRIPTION OF DRAWINGS

The invention can be more fully understood by reading the subsequent detailed description and examples with references made to the accompanying drawings, wherein:
FIG. 1 is a diagram of a physiological signal processing circuit according to an embodiment of the invention;
FIG. 2 is a diagram of a processing circuit according to an embodiment of the invention;
FIG. 3A is a diagram of a waveform of the digital signal according to an embodiment of the invention;
FIG. 3B is a diagram of waveforms of a filtered signal and an intermediate signal according to an embodiment of the invention;
FIG. 3C is a diagram of the selection and generation of an output signal according to an embodiment of the invention;
FIG. 4 is a diagram of a wearable device according to an embodiment of the invention; and
FIG. 5 is a flowchart of a method for processing physiological signals according to an embodiment of the invention.

### DETAILED DESCRIPTION OF THE INVENTION

In order to illustrate the purposes, features and advantages of the invention, the embodiments and figures of the invention are shown in detail as follows.

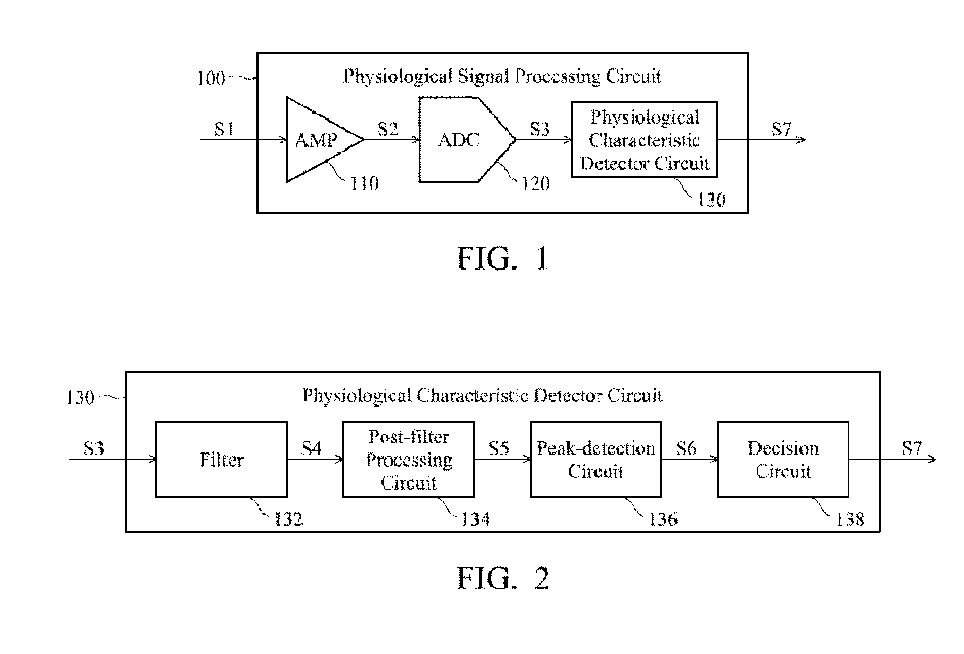

FIG. 1 is a diagram of a physiological signal processing circuit 100 according to an embodiment of the invention. As shown in FIG. 1, the physiological signal processing circuit 100 at least includes an amplifier (AMP) 110, an analog-to-digital converter (ADC) 120, and a physiological characteristic detector circuit 130. The physiological signal processing circuit 100 may be an independent integrated circuit (IC) chip implemented in a mobile device, such as a smartphone, a tablet computer, a notebook computer, or a wearable device. The amplifier 110 is configured to amplify an analog physiological signal S1 of a user for providing an amplified signal S2. The analog physiological signal S1 may be a natural signal related to a human body, such as a heartbeat, a pulse, or a blood pressure, and it may have been preprocessed by other circuits. For example, the analog physiological signal S1 may be a photoplethysmogram (PPG) signal or an electrocardiography (ECG) signal. The analog-to-digital converter 120 is coupled to the amplifier 110, and is configured to perform a sampling process and convert the amplified signal S2 to a digital signal S3. For example, the digital signal S3 may include raw data, such as multiple bits which represent time and amplitude of heartbeats. The physiological characteristic detector circuit 130 is coupled to the analog-to-digital converter 120, and is configured to detect a physiological characteristic of the user from the digital signal S3 so as to provide an output signal S7. The output signal S7 is informative of a physiological characteristic of the user. For example, the physiological characteristic of the user may be one of a heart rate, a heart beat interval, and a heart beat instant. The output signal S7 may, for example, also include one of a period between two successive heartbeats and a specific time instant with respect to a heartbeat.. In addition, the period between two adjacent heartbeats may be further converted to heart rate by using a divider (not shown). Since the output signal S7 is generated by processing the digital signal S3, the amount of data that needs to be transmitted in the output signal S7 is significantly reduced, and the data rate of the output signal S7 is much lower than the data rate of the unprocessed digital signal S3. The physiological signal processing circuit 100 can transmit the output signal S7, rather than the original digital signal S3 including the raw data, to other external devices, such that the required data rate and power consumption can be effectively improved. The physiological characteristic detector circuit 130 of the physiological signal processing circuit 100 can help to reduce the computational burden on the external devices. An example of the external devices may be a processor. On the one hand, the processor might consume less power with lower data rate. On the other hand, with the physiological characteristic detector circuit 130 takes some job from the processor, the processor might enter into some sleep mode to save more power. The detailed operation of the physiological signal processing circuit 100 will be described in the following figures and embodiments. It should be understood these embodiments are just exemplary, and they are not used to limit the scope of the invention.

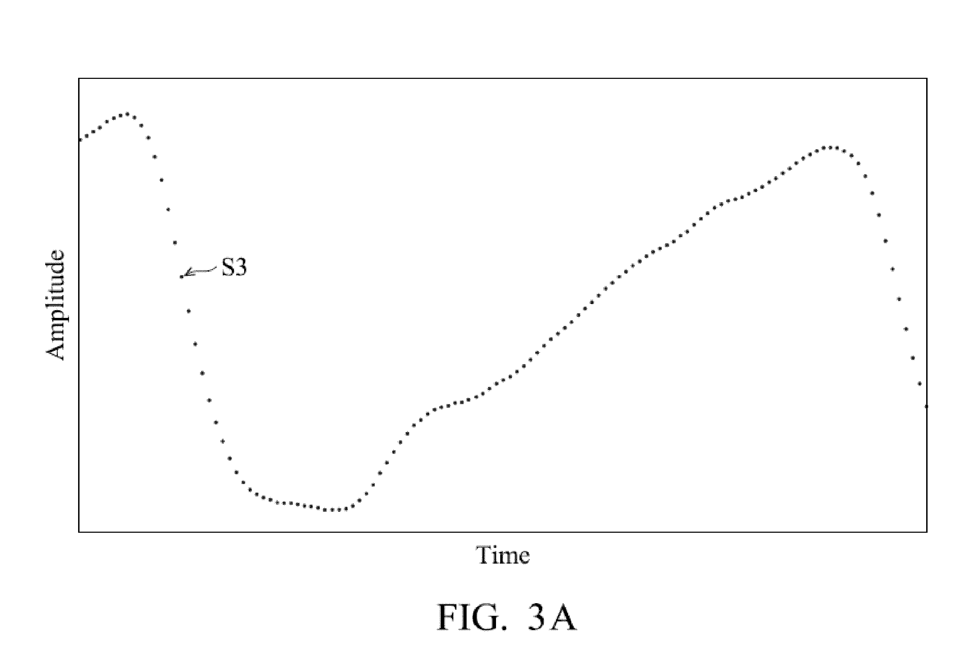

FIG. 2 is a diagram of the physiological characteristic detector circuit 130 according to an embodiment of the invention. In the embodiment of FIG. 2, the physiological characteristic detector circuit 130 includes one or more of the following components: a filter 132, a post-filter processing circuit 134, a peak-detection circuit 136, and a decision circuit 138. FIG. 3A is a diagram of a waveform of the digital signal S3 according to an embodiment of the invention. After the analog physiological signal S1 is amplified and digitalized, the generated digital signal S3 includes raw data related to physiological information from a human body. For example, if the analog physiological signal S1 is a photoplethysmogram (PPG) signal or an electrocardiography (ECG) signal, the raw data of the digital signal S3 may include many bits which represent time domain waveform, consisting of direct current (DC) magnitude and alternating current (AC) magnitude of heartbeats of the human body being monitored.

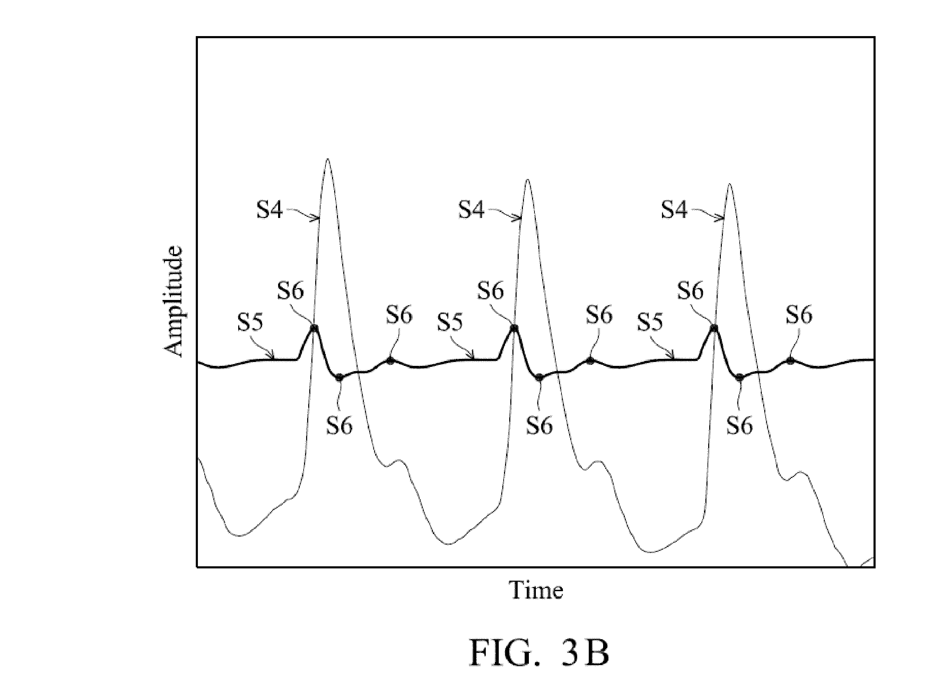

The filter 132 is configured to filter the digital signal S3 and provide a filtered signal S4 in a first signal domain. For example, the filter 132 may be implemented with a (digital) low-pass filter, and the filtered signal S4 may include only the low-frequency components of the digital signal S3. For example, the filter 132 may be implemented with a combination of a low-pass filter and a high-pass filter, and the filtered signal S4 may include only the mid-frequency components of the digital signal S3. The first signal domain may be a first time domain which includes information of signal amplitude. The low-pass filter can remove high-frequency noise. The high-pass filter can remove low-frequency DC variation, and reduce the number of bits of signals. For example, if the digital signal S3 has 16 bits, the filtered signal S4 may have only 12 bits. The post-filter processing circuit 134 is configured to process the filtered signal S4 and provide an intermediate signal S5 in a second signal domain. For example, the post-filter processing circuit 134 may be implemented with a differential unit, and the intermediate signal S5 may include a first derivative or a second derivative of the filtered signal S4. The second signal domain may be a second time domain which includes information of signal slope, signal maximum points, signal minimum points, and/or signal absolute values. FIG. 3B is a diagram of waveforms of the filtered signal S4 and the intermediate signal S5 according to an embodiment of the invention. It should be understood that the waveforms of the filtered signal S4 and the intermediate signal S5 are digital and discrete in fact, and they are presented in an analog and continuous manner for the reader to more easily comprehend. In the embodiment of FIG. 3B, the intermediate signal S5 is a first derivative of the filtered signal S4. In alternative embodiments, adjustments are made such that intermediate signal S5 is an absolute value of a first derivative or a second derivative of the filtered signal S4.

The peak-detection circuit 136 is configured to detect local peak values among the intermediate signal S5 and for providing multiple data points S6. Please refer to FIG. 3B. Each data point S6 may be equivalent to a respective local maximum or minimum point of the intermediate signal S5. Generally, the local maximum points of the intermediate signal S5 may represent systolic points of heart, and these points may be collected by the peak-detection circuit 136 so as to form the data points S6.

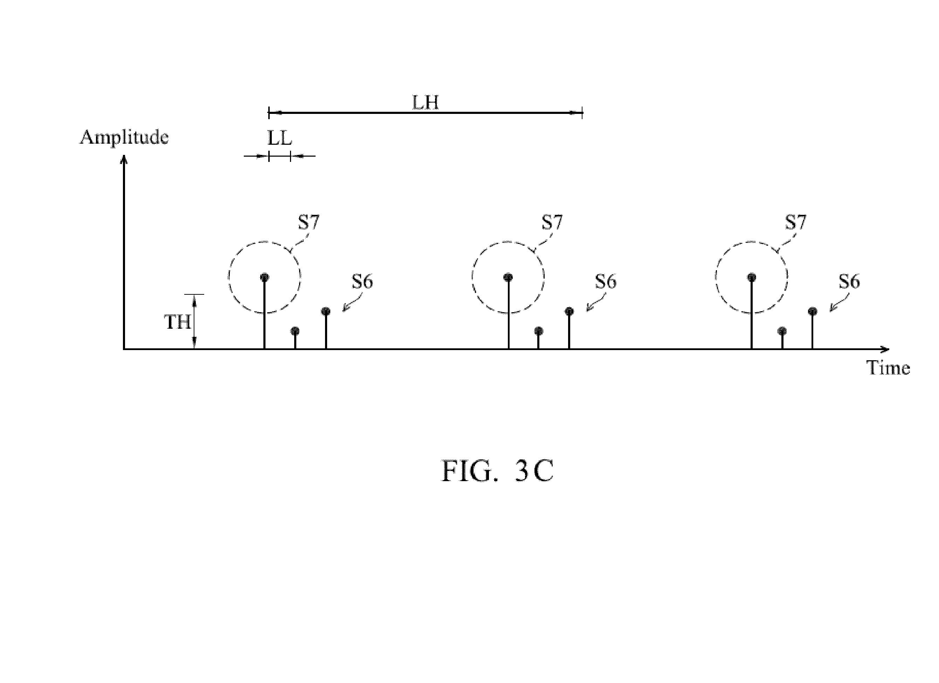

The decision circuit 138 is configured to generate the output signal S7 by picking up the data points S6 according to a decision rule. The output signal S7 may include only the picked data points S6. For example, the decision circuit 138 may select some of the data points S6 as multiple heartbeat points according to the decision rule, and the heartbeat points may be used to produce the output signal S7. In some embodiments, the information of picked heartbeat points is combined with its corresponding time information, such that the time intervals between every two data point S6 can be calculated. FIG. 3C is a diagram of the selection and generation of the output signal S7 according to an embodiment of the invention. In the embodiment of FIG. 3C, the selection process and the decision rule of the decision circuit 138 include the operations of: (1) determining whether a respective data point S6 is higher than a threshold value TH; (2) determining whether a respective interval between two adjacent data points S6 is longer than the shortest reasonable length LL; and (3) determining whether a respective interval between two adjacent data points S6 is shorter than the longest reasonable length LH. If the aforementioned conditions (1), (2), and (3) are all satisfied, the corresponding data point(s) of the corresponding data point(s) S6 will be determined to have passed the pick-up process and will be selected as a heartbeat point (i.e., a picked data point) so as to produce the output signal S7. Otherwise, the corresponding data point(s) S6 will be abandoned and not form any part of the output signal S7. The decision circuit 138 is used to remove obviously unreasonable data points S6. For example, since the heart rate of a normal human being has an upper boundary of about 200 beats per minute, an interval which is smaller than 0.3 seconds between two adjacent data points S6 is obviously unreasonable, and the two adjacent data points are required to be picked up again, or just abandoned. Furthermore, the corresponding S6 with regard to output signal S7 may be used to update the threshold value TH. For instance, as the magnitude of S6 increases, the threshold value TH may be updated; this may be formulated as THnew= THcur+ (magS6- THcur)*alpha, where THnew is the updated TH value, THcur is the current TH, magS6 is the magnitude of the corresponding S6 with regard to the latest data point of output signal S7 and alpha is a scaling factor, e.g. 0.5. This is because the amplitude of the digital signal S3 may vary from time to time because of some environmental changes. As the amplitude of digital signal S3 increases, the amplitude of the output signal S6 increases as well; therefore a fixed threshold value TH may yield poor performance in some cases.

In alternative embodiments, the filter 132 and the post-filter processing circuit 134 are combined into a single filter, and the filtered signal S4, the intermediate signal S5, and the data points S6 are deemed to be a single inner signal.

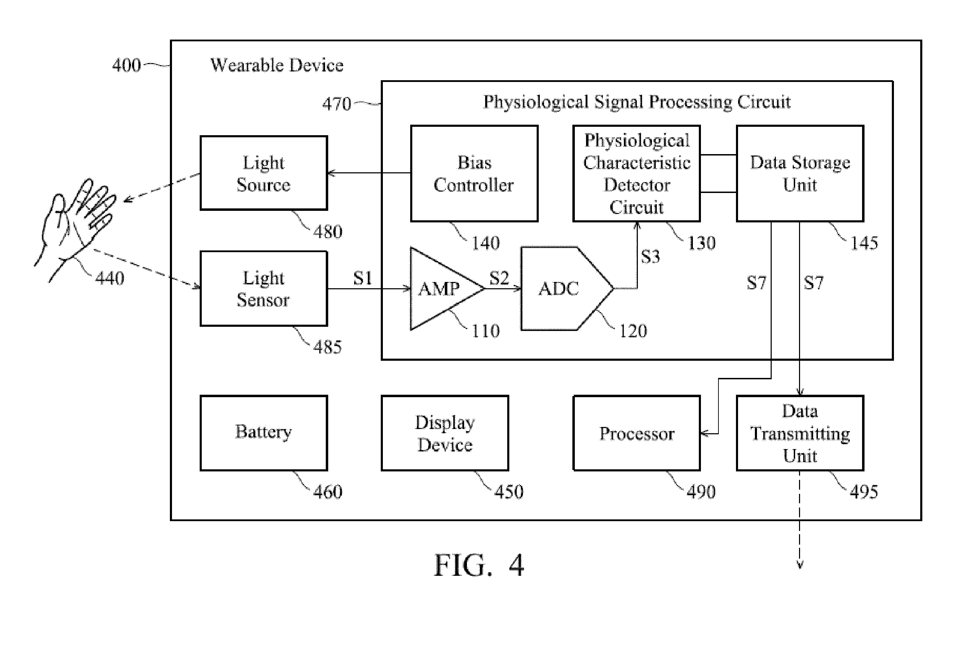

FIG. 4 is a diagram of a wearable device 400 according to an embodiment of the invention. The type of wearable device 400 is not limited in the invention. For example, the wearable device 400 may be a smart watch or a sports wristband for use on the human body 440. In the embodiment of FIG. 4, the wearable device 400 includes one or more of the following components: a display device 450, a battery 460, a physiological signal processing circuit 470, a light source 480, a light sensor 485, a processor 490, and a data transmitting unit 495. The display device 450 may be a liquid-crystal display (LCD). The battery 460 is configured to supply electric power to every component in the wearable device 400. The detailed structure and operation of the physiological signal processing circuit 470 have been described in the embodiments of FIG. 1, 2, and 3A-3C, as the physiological signal processing circuit 100. The difference from the above embodiments is that the physiological signal processing circuit 470 further includes a bias controller 140. Note that the physiological signal processing circuit 470 might be fabricated on a discrete IC or integrated with other components listed in FIG. 4. In some embodiments, the physiological characteristic detector circuit 130 is further coupled to a data storage unit 145, such as static random access memory (SRAM) or dynamic random access memory (DRAM). The data storage unit 145 is optional and used to temporarily store the output signal S7. The processor 490 is triggered to process the output signal S7 stored in the data storage unit 145 when a trigger condition occurs. In some embodiments, the processor 490 monitors a capacity of the data storage unit 145 periodically (e.g., every 1 minute), and reads the data stored in the data storage unit 145 when the capacity of the data storage unit 145 is smaller than a predetermined value. In alternative embodiments, the processor 490 reads the data stored in the data storage unit 145 at a specific frequency (e.g., every 3 minutes). In other embodiments, the processor 490 reads the data stored in the data storage unit 145 when the data storage unit 145 notifies the processor 490.

The light source 480 is controlled by the bias controller 140 and configured to emit light to the human body 440. For example, the light source 480 may include a light-emitting diode (LED) for generating the light at a predetermined frequency. In response, the light sensor 485 is configured to receive reflection or transmission light from the human body 440 and generate an analog physiological signal S1. For example, transmission light through the human body 440 (e.g., a finger or wrist) may have a relatively strong intensity during the systole phase of the cardiac cycle, and a relatively weak intensity during the diastole phase. The transmission light may be detected by the light sensor 485 so as to form a photoplethysmogram (PPG) signal (the analog physiological signal S1). The physiological signal processing circuit 470 is configured to process the analog physiological signal S1 from the light sensor 485 and generate the output signal S7. The processor 490 may be independent of the physiological signal processing circuit 470 and configured to further process the output signal S7 from the physiological signal processing circuit 470. For example, the processor 490 may derive the physiological characteristic of the user (human body 440) according to the output signal S7. The data transmitting unit 495 is coupled to the physiological characteristic detector circuit 130 of the physiological signal processing circuit 470, and configured to transmit the output signal S7 to an external device (not shown) through a wired or wireless communication link. For example, the wired communication link may include an inter-integrated circuit (I2C) bus or a service provider interface (SPI), and the wireless communication link may include a Bluetooth or Wi-Fi wireless connection. When the wearable device 400 is implemented with a smart watch, it can detect physiological signals from a user and transmit the processed digital signal to an external device, such that the external device can interact with the user in a variety of ways. For example, the external device may be used as a sleep monitor or for an examination of the user's health by collecting necessary information from the wearable device 400. Since the wearable device 400 only transmits an output signal S7 that has been processed, the amount of data transmission between the wearable device 400 and its related external device is significantly reduced, and the power consumption of the whole system is also improved. In addition, with the processor 490 having to do less computation, it might be turned off or enter into a sleep mode to save more power.

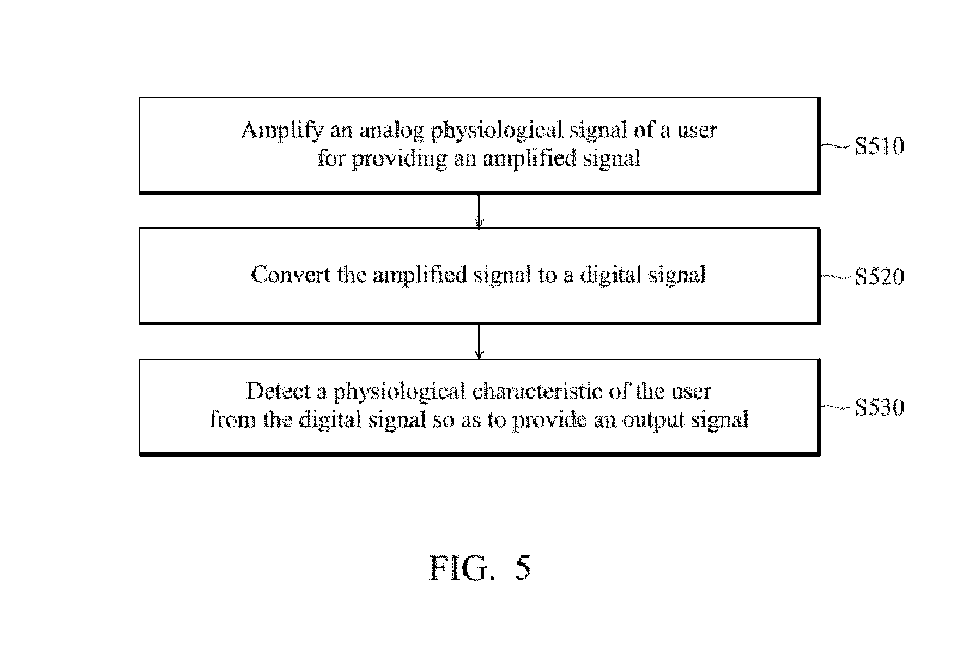

FIG. 5 is a flowchart of a method for processing physiological signals according to an embodiment of the invention. In step S510, an analog physiological signal of a user is amplified for providing an amplified signal. In step S520, the amplified signal is converted to a digital signal. In step S530, a physiological characteristic of the user is detected from the digital signal so as to provide an output signal. It should be understood that the above steps are not required to be performed in order, and any one or more features of the embodiments of FIGS. 1-4 may be applied to the method of FIG. 5.

The physiological signal processing circuit of the invention includes the processing circuit, and therefore it can process complex raw data (i.e., the digital signal) and then merely transmit the processed data (i.e., the output signal). With such a design, the amount of data to be transmitted and the power consumption of the whole system are both effectively improved. For example, if the sampling rate of the analog-to-digital converter is 125Hz and every sample point is recorded with 22 bits, without being processed by the processing circuit, the required rate of data transmission will be 2750 (125 × 22 = 2750) bits per second. However, if the invention is used and only the processed data are transmitted, with the assumption that the heart rate of a human body is, at most, 200 beats per minute and every heartbeat is recorded with 24 bits, the required rate of data transmission will be merely 80 (200 ÷ 60 × 24 = 80) bits per second. In other words, by using the invention, the data rate of the processed data (i.e., the output signal) is less than 0.03 times the data rate of the raw data (i.e., the digital signal). The physiological signal processing circuit of the invention at least has the advantage of reducing the amount of data transmission, reducing memory usage, and reducing the computation and power consumption of the processor. Therefore, the invention is suitable for application in many mobile electronic devices which include a limited-power battery.

The method of the invention, or certain aspects or portions thereof, may take the form of program code (i.e., executable instructions) embodied in tangible media, such as floppy diskettes, CD-ROMS, hard drives, or any other machine-readable storage medium, wherein, when the program code is loaded into and executed by a machine such as a computer, the machine thereby becomes an apparatus for practicing the methods. The methods may also be embodied in the form of program code transmitted over some transmission medium, such as electrical wiring or cabling, through fiber optics, or via any other form of transmission, wherein, when the program code is received and loaded into and executed by a machine such as a computer, the machine becomes an apparatus for practicing the disclosed methods. When implemented on a general-purpose processor, the program code combines with the processor to provide a unique apparatus that operates analogously to application-specific logic circuits.

Use of ordinal terms such as "first", "second", "third", etc., in the claims to modify a claim element does not by itself connote any priority, precedence, or order of one claim element over another or the temporal order in which acts of a method are performed, but are used merely as labels to distinguish one claim element having a certain name from another element having the same name (but for use of the ordinal term) to distinguish the claim elements.

While the invention has been described by way of example and in terms of the preferred embodiments, it is to be understood that the invention is not limited to the disclosed embodiments. On the contrary, it is intended to cover various modifications and similar arrangements (as would be apparent to those skilled in the art). Therefore, the scope of the appended claims should be accorded the broadest interpretation so as to encompass all such modifications and similar arrangements.

## Claims

1. A physiological signal processing circuit, comprising:
an amplifier, amplifying an analog physiological signal of a user for providing an amplified signal;
an analog-to-digital converter, coupled to the amplifier, and converting the amplified signal to a digital signal; and
a physiological characteristic detector circuit, coupled to the analog-to-digital converter, for detecting a physiological characteristic of the user from the digital signal so as to provide an output signal.

2. The physiological signal processing circuit as claimed in claim 1, wherein the analog physiological signal is a photoplethysmogram (PPG) signal or an electrocardiography (ECG) signal.

3. The physiological signal processing circuit as claimed in claim 2, wherein the output signal comprises one of a period between two successive heartbeats and a specific time instant with respect to a heartbeat.

4. The physiological signal processing circuit as claimed in claim 1, wherein a data rate of the output signal is lower than a data rate of the digital signal.

5. The physiological signal processing circuit as claimed in claim 1, wherein the physiological characteristic of the user is one of a heart rate, a heart beat interval, and a heart beat instant.

6. The physiological signal processing circuit as claimed in claim 1, further comprising:
a processor; and
a data storage unit for storing the output signal, wherein the processor is triggered to process the output signal stored in the data storage unit when a trigger condition occurs.

7. The physiological signal processing circuit as claimed in claim 1, wherein the physiological characteristic detector circuit comprises:
a filter, filtering the digital signal to provide a filtered signal in a first signal domain; and
a post-filter processing circuit, process the filtered signal to provide an intermediate signal in a second signal domain.

8. The physiological signal processing circuit as claimed in claim 7, wherein the physiological characteristic detector further comprises:
a peak detection circuit, detecting local peak values among the intermediate signal for providing a plurality of data points; and
a decision circuit, selecting some of the plurality of data points as a plurality of heartbeat points, wherein the plurality of heartbeat points are used to produce the output signal.

9. The physiological signal processing circuit as claimed in claim 1, wherein the physiological characteristic detector circuit is further coupled to a data transmitting unit for transmitting the output signal to a device through a wired or wireless communication link.

10. The physiological signal processing circuit as claimed in claim 1, wherein a data rate of the output signal is less than 0.03 times of a data rate of the digital signal.

11. A method for processing physiological signals, comprising the steps of:
amplifying an analog physiological signal of a user for providing an amplified signal;
converting the amplified signal to a digital signal; and
detecting a physiological characteristic of the user from the digital signal so as to provide an output signal.

12. The method as claimed in claim 11, wherein the analog physiological signal is a photoplethysmogram (PPG) signal or an electrocardiography (ECG) signal.

13. The method as claimed in claim 11, wherein the output signal informs one of a period between two adjacent heartbeats and a specific time instant with respect to a heartbeat.

14. The method as claimed in claim 11, wherein a data rate of the output signal is lower than a data rate of the digital signal.

15. The method as claimed in claim 11, wherein the physiological characteristic of the user is one of a heart rate, a heart beat interval, and a heart beat instant.
